# EUROPEAN PATENT APPLICATION

(11) **EP 3 028 666 A1**
(43) Date of publication of application: **08.06.2016**
(21) Application number: 14195629.2
(22) Date of filing: 01.12.2014
(51) Int. Cl.: A61F 2/00

(54) **RESORBABLE MEDICAL MESH IMPLANT WITH MECHANICAL CHARACTERISTICS VARYING ALONG ITS WIDTH**

(71) Applicant: Novus Scientific AB, 754 50 Uppsala (SE)
(72) Inventor: Egnelöv, Per, 754 40 Uppsala (SE)
(74) Representative: Brann AB

(57) **Abstract**

The invention relates to a degradable medical implant (11), comprising an essentially two-dimensional mesh with a width (W) and a modulus of elasticity (E), for reinforcing abdominal wall tissue at and around an incisional wound. The mesh is characterized in that the modulus of elasticity (E) varies continuously or gradually over the width (W) of the two-dimensional mesh.

## Description

### Field of the Invention

The present invention relates to a medical mesh device for supporting and reinforcing wounded soft tissue, and in particular to a resorbable medical mesh implant for supporting and reinforcing abdominal wall tissue after an invasive medical operation which has involved the opening of the abdominal wall, and even more particularly to a resorbable medical mesh implant which has a modulus of elasticity which varies over the width of the mesh implant.

### Background of the Invention

Within the area of medical surgery, there are a large number of medical operations and treatments that require the opening of the abdominal wall of a human patient. When the primary medical surgery or treatment has been completed, the opening in the patient's abdominal wall has to be closed. This last procedure is normally accomplished by means of a suture which is sutured across the incisional wound in the abdominal wall. To actually close the abdominal wound or opening, considerable tension has to be applied in the suture, which leads to a risk that the suture ruptures through the perforated, stitched tissue around the abdominal wound or opening. This post-surgical complication - which commonly is referred to as wound rupture and typically appears within days or a week after the primary surgery - constitutes a very serious medical condition, which involves the risk that the patient is lost or that his/her planned medical treatment cannot be pursued. Another medical complication that can occur is so-called incisional hernia, which implies that a patient's intestines bulge out at the place of the scar which followed from the surgery. This situation can occur months or even years after the primary surgery and is many times associated with degradable sutures.

To eliminate or at least reduce the impact of the complications mentioned above, a doctor can choose to use a medical mesh when closing the abdominal wound or opening. Such a mesh is implanted over the suture line as an extra support and reinforcement to relieve the tension in the suture and, in the case of a degradable suture, it can remain in the patient's body also after the suture has lost its mechanical integrity. Medical meshes used in these areas are medical standard meshes which are commercially available under trademarks such as Prolene^{™}, which is a nonabsorbable (non-degradable) polypropylene mesh; Mersilene™, which is a nonabsorbable polyester mesh; Ultrapro™, which is a partly absorbable mesh made from ε-caprolactone, glycolide and polypropylene; and Vicryl™, which is an absorbable mesh made from glycolide and lactide.

Although the implantation of such a mesh at the site of an abdominal wound or opening does reduce the risk of medical complications such as wound rupture and incisional hernia, the commercially available meshes are typically designed to be applicable in a variety of medical situations and can be therefore be regarded as universal meshes. In other words, these commonly used meshes are not tailored and optimized for, e.g., the load situation prevailing at the site of a sutured abdominal wound. Thus, there is still a need for an improved medical mesh whose strength profile is adapted to the load situation prevailing at the position of an incisional wound created in the abdominal wall of a human patient. Preferably, an improved medical mesh should also stimulate regeneration of new healthy bodily tissue, and should also provide for good integration into the body's own tissue.

### Summary of the Invention

The above-mentioned objects are achieved by the present invention according to the independent claim. Preferred embodiments are set forth in the dependent claims.

A medical mesh device according to the present invention is intended to be implanted at the site of an incisional wound, which results from a previously performed surgery in the abdominal region of a human patient; and the medical mesh is in particular intended and designed to be positioned over a suture line, which has been applied by a medically trained person in order to close the surgical wound. The medical mesh will thereby act as an extra reinforcement, which reduces the tension in the suture or sutures, which, in turn, leads to less risk of wound rupture. By remaining in the body also after the point in time when a degradable suture has lost its mechanical integrity and also being capable of stimulating the regeneration of new bodily tissue, a medical mesh according to the present invention can contribute to reducing the risk that an incisional hernia is developed at a later stage of a patient's life time. In one embodiment, a rectangular medical mesh exhibits a strength profile which, over the width of the mesh, gradually transits from a strong and inelastic midsection to more elastic and mechanically compliant rim sections. In another embodiment, a rectangular medical mesh device exhibits a strength profile which, over the width of the mesh, continuously transits from a strong and inelastic midsection to more elastic and mechanically compliant rim sections. Meshes according to the present invention are preferably synthetic meshes, which preferably are made from biodegradable polymers that degrade in a patient's body.

### Brief Description of the Drawings

Fig. 1a shows a schematic picture of a patient's body with a partly open abdominal region, in which an incision has been created and closed by a line of sutures sutured in the aponeuroses, and Fig. 1b shows the patient of Fig. 1a with a medical mesh according to the invention positioned and fixated over most of the suture line.
Fig. 2a shows a schematic view of a first embodiment of a rectangular medical mesh according to the present invention, and Fig. 2b shows the accompanying stepwise diagram of modulus of elasticity (E) versus mesh width (W).
Fig. 3a shows a schematic view of a second embodiment of a rectangular medical mesh according to the present invention, and Fig. 3b shows the accompanying continuous diagram of modulus of elasticity (E) versus mesh width (W).

### Detailed Description of Preferred Embodiments

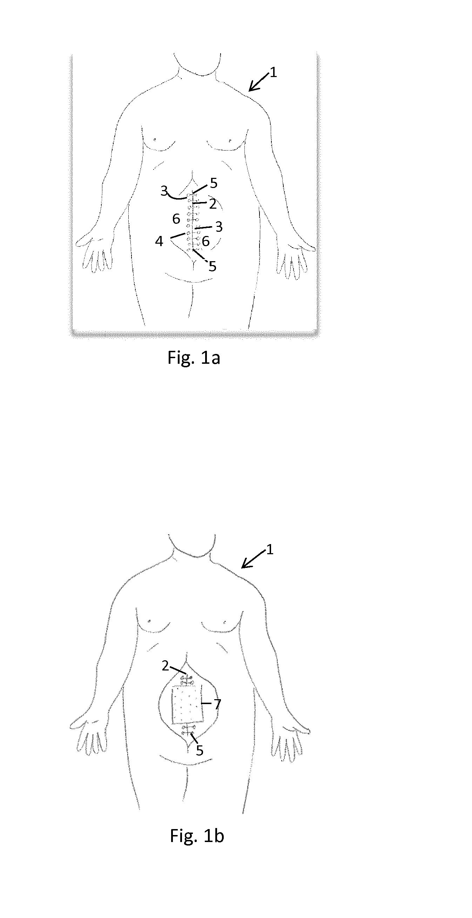

The inventor of the present invention has realized that the abdominal tissue region at and around an incisional wound, which has been created by a medical doctor in order to perform a medical operation or treatment in the abdominal cavity of a human patient and which subsequently has been closed by suturing, exhibits a varying strength profile. This situation is schematically depicted in Fig. 1a, wherein a human body 1 has an incisional wound 2 in partly exposed subcutaneous tissue in the abdomen. From Fig. 1a it is further discernible that the aponeuroses in the abdominal regions 3 on both sides of the incisional wound 2 have been perforated by a needle (not seen in the figure) which has left holes 4, through which sutures 5 have been sewn in order to close the incisional wound 2. The subcutaneous tissues in this patient's abdomen will therefore exhibit very different mechanical properties depending on their positions relative the incisional wound 2. That is, distant tissue regions 6, which are located outside (as seen from the incisional wound 2) of regions 3, are virtually unaffected by the surgery performed and have more or less normal mechanical strengths, whereas the incisional wound 2 itself has virtually no mechanical strength, and the intermediate regions 3 have a mechanical strength which is somewhere between these two extremes. A medical mesh according to the present invention is adapted and tailored to the situation described above, by having a strength profile which is matched (or, more accurately, counter-matched) to the strengths of the tissue regions surrounding an incisional wound in an abdominal wall.

Fig. 1b shows the human body 1 of Fig. 1a, which now has got a medical implant 7, herein also referred to as a medical mesh implant, according to the present invention placed and fixated over most of the sutures 5 in the aponeuroses. The medical mesh implant 7 will thereby serve as an extra reinforcement, which reduces the tension in the sutures 5, which, in turn, leads to less risk of rupture of the incisional wound 2. By remaining in the body also after the point in time when a degradable suture has lost its mechanical integrity and also being capable of stimulating the regeneration of bodily tissue, a medical mesh implant according to the present invention can contribute to reducing the risk that an incisional hernia is developed at a later stage of a patient's life time.

Further, as will be thoroughly explained below, the medical mesh implant 7 has a strength profile which is counter-matched to the overall strength profile of the abdominal region of the patient 1. More precisely, the medical mesh implant 7 has a midsection with high mechanical strength (which, when implanted, is located over the very weak incisional wound 2), intermediate sections with moderate mechanical strengths (which, when implanted, are located over the fairly weak intermediate regions 3), and outer sections with the lowest mechanical strength (which, when implanted, are located over the strong distant regions 6). It should therefore be appreciated that the medical mesh implant 7 is not excessively and unnecessarily strong in its intermediate and outer sections. Since it is known that damaged soft tissue regenerates and heals best when it is exposed to mechanical load (by a process known as mechanotransduction), this gradual decrease in mechanical strength promotes the body's own healing process and contributes to the creation of new and healthy soft tissue.

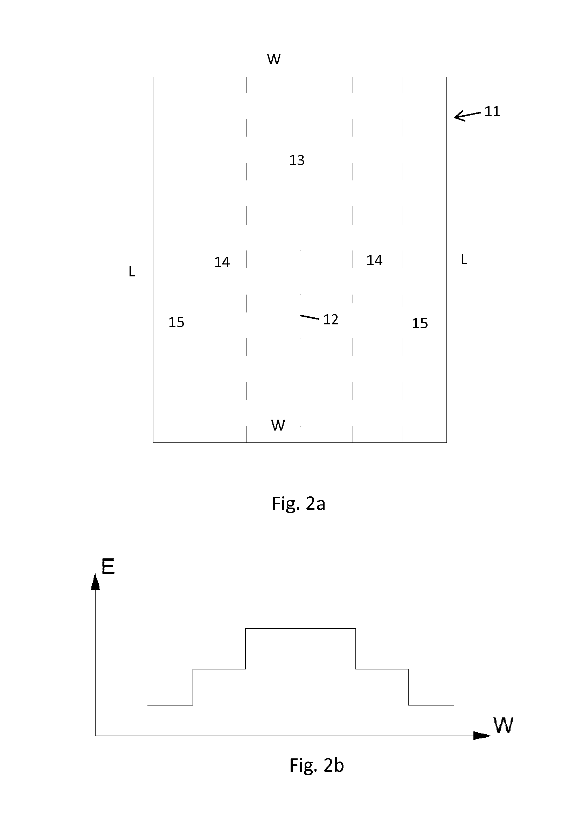

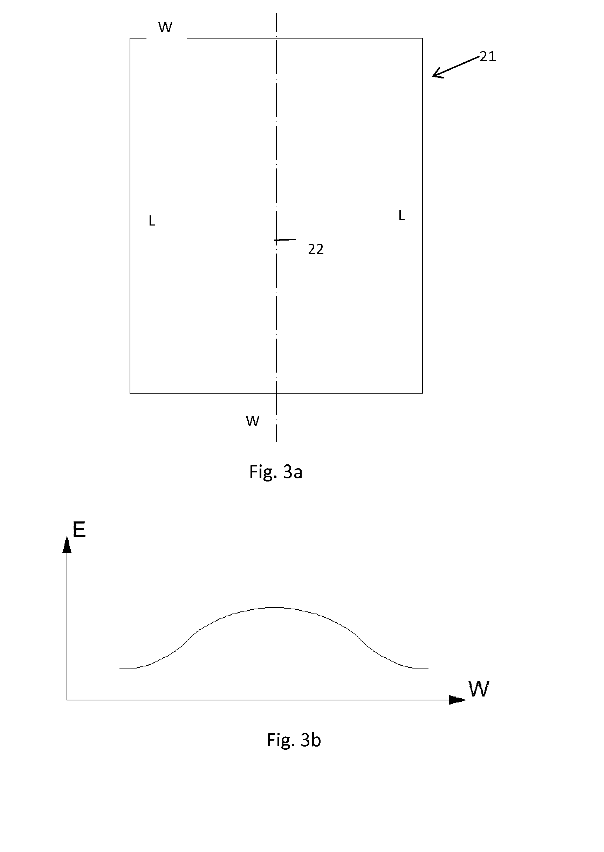

Fig. 2a shows a first embodiment of a medical mesh implant 7, consisting of a mesh 11 according to the present invention. The mesh 11, which is a thin, or substantially flat, or essentially two-dimensional object or structure, has a rectangular shape, with a length L and a width W, and a midline 12. The length L should be long enough the cover the length of any incisional opening created in the abdominal wall of a patient, and can, for example, be between 20 cm and 50 cm. The width W should be large enough to cover all regions that may have been damaged or weakened by the creation of an incisional opening in the abdominal wall and should preferably also be large enough to cover some of the unaffected tissue regions on both sides of an incisional wound, and can, for example, be between 10 cm and 30 cm. The mesh 11 has been divided into five (5) sections over its width, i.e. one midsection and two further sections on each side of a midline 12. Here it can be mentioned that all the dashed lines in Fig. 2a (and also in Fig. 3a below) have merely been introduced as guides for the eye, and may also be referred to as imaginary lines. More precisely, the mesh 11 has one (1) midsection 13, two (2) intermediate sections 14, and two (2) outer edge or rim sections 15. Fig. 2b illustrates the diagram of modulus of elasticity (E) versus the width (W) for the mesh 11, and it can be appreciated that the midsection 13 is characterized by having a high modulus of elasticity, that intermediate sections 14 are characterized by having a moderately high modulus of elasticity, and that outer rim or edge sections 15 are characterized by having a low modulus of elasticity. The modulus of elasticity of the rim or edge sections 15 may suitably be greater than or equal to 10% and equal to or less than 30% (such as 10, 15, 20, 25, or 30 %) of the modulus of elasticity of the midsection 13. The modulus of elasticity of the intermediate sections 14 may suitably be greater than or equal to 30% and equal to or less than 70% (such as 30, 40, 50, 60, or 70 %) of the modulus of elasticity of the midsection 13. Fig. 2b depicts a change in modulus of elasticity E of the same magnitude at the transition from each section to the next section. However, alternatively, the change in modulus of elasticity E may be of different magnitude at different section transitions (not shown). As should be clear from the discussion above in conjunction with Fig. 1, the purpose of the respective strengths of these different sections 13, 14, 15 is to counter-match the strengths of the abdominal wall sections at and around an incisional wound. In other words, once implanted, mesh sections 13, 14 and 15, respectively, are strong where underlying bodily sections are weak, and vice versa.

As is evident from Fig. 2b, the change in elasticity from one section to a neighbouring section is abrupt for the mesh 11. This feature implies that corresponding physical transition sections in the mesh 11 are exposed to large strains when forces are acting on the implanted mesh. Therefore, another embodiment of a medical mesh implant 7, consisting of a mesh 21 according to the present invention is described in Fig. 3a and Fig. 3b. As seen in Fig. 3a, the mesh 21 is also an essentially two-dimensional object with a midline 22 and a length L and a width W, with the same preferred length and width intervals as mesh 11 described above. The mesh 21 is, however, not divided into a number of different sections having very different moduli of elasticity. From Fig. 3b, it is evident that the mesh 21 is instead characterized by having a modulus of elasticity (E) which is high in the center of the mesh 21 and which continuously decreases towards the opposing edges of its width. The modulus of elasticity of the edges of the mesh 21 may suitably be greater than or equal to 10% and equal to or less than 30% (such as 10, 15, 20, 25, or 30 %) of the modulus of elasticity of the center of the mesh 21. The modulus of elasticity of the section between each edge and the center may suitably be greater than or equal to 30% and equal to or less than 70% (such as 30, 40, 50, 60, or 70 %) of the modulus of elasticity of the center of the mesh 21.

The two embodiments of a medical mesh implant that have been disclosed and discussed above can be regarded as extremes of the present invention. That is, while the mesh 11 exhibits a very pronounced gradual decrease in modulus of elasticity from a midsection to a rim section, with only one intermediate section interposed therebetween, more intermediate sections with successively less strength could be provided. Such meshes would exhibit a less pronounced gradual decrease in modulus of elasticity, and their corresponding diagrams of modulus of elasticity versus width would more resemble the continuous diagram of elasticity versus width shown for mesh 21. Just to complete this discussion, also a mesh having only two different levels of modulus of elasticity would fall within the scope of the invention. Herein, the terms "gradual" and "stepwise" have the same meaning, and refer to a sudden, more or less instant change in modulus of elasticity. A medical mesh implant according to the present invention can therefore have a gradually changing modulus of elasticity or a continuously changing modulus of elasticity. Further, meshes and mesh structures described herein have been referred to as essentially two-dimensional objects. This means merely that a mesh structure according to the invention has a thickness which is very small in comparison with its length or width, but it does not mean that the thickness is negligible as such; and indeed, one way of affecting the modulus of elasticity is to provide a thicker mesh structure, e.g., more layers of mesh, at some sections of a mesh structure.

Different techniques can be utilized to produce a medical mesh implant according to the present invention. For example, in knitting or weaving techniques, knitting or weaving patterns can be changed from one section to another or be continuously changed, e.g. by varying pore sizes or number of fibers per surface area or include more fibers with higher modulus of elasticity. It is also possible to sew or otherwise join together different mesh sections having different moduli of elasticity, or even at some sections to arrange two or more layers of mesh on top of each other. During manufacturing of medical meshes, which are intended to be implanted in a human body, the meshes are usually heat treated. This process, which in the art also is referred to as annealing, means that mesh material is exposed to heat. To produce sections with different mechanical strength, certain sections of the mesh material can be exposed to more heat, i.e. higher temperature or longer dwell time. By the different manufacturing methods discussed above, a mesh structure according to the invention can, for example, have a highest modulus of elasticity of 3 GPa (3x10⁹ Pascal) and a lowest modulus of elasticity of 300 kPa (3x10⁵ Pascal), and can, depending on the design, continuously or gradually transit from these extreme values, as has been described above. The modulus of elasticity E may be measured using the ASTM D3787-1 guideline for ball burst strength, as described in EP 2 002 800 B1.

To fully exploit the advantageous effect of a medical mesh implant according to the present invention, it is believed that the medical mesh implant should be a biodegradable mesh implant that degrades within a human body. Further, a mesh should preferably be a synthetic mesh, since it is believed that the moduli of elasticity are easier to control and to dimension for a synthetic mesh than for, e.g., a biological product. Non-limiting examples of polymers suitable for the meshes presented herein are synthetic resorbable polymers made from the monomers glycolide, lactide, and all stereoisomers thereof; trimethylene carbonate, ε-caprolactone, dioxanone or dioxepanone, or various combination thereof. Yet other non-limiting examples of synthetic resorbable polymers that can be utilized are various aliphatic polyurethanes, such as polyureaurethanes, polyesterurethanes and polycarbonateurethanes, and also materials such as polyphosphazenes or polyorthoesters.

Although the present invention has been described with reference to specific embodiments, also shown in the appended drawings, it will be apparent to those skilled in the art that many variations and modifications can be done within the scope of the invention as described in the specification and defined with reference to the claims below.

## Claims

1. A degradable medical implant, comprising an essentially two-dimensional mesh (11; 21), which has a width (W) and a modulus of elasticity (E), for reinforcing abdominal wall tissue at and around an incisional wound, **characterized in that** the modulus of elasticity (E) varies over the width (W) of the essentially two-dimensional mesh (11; 21).

2. The degradable medical implant according to claim 1, wherein the essentially two-dimensional mesh (11; 21) has an imaginary midline (12; 22), which bisects the width (W), and that the modulus of elasticity (E) varies symmetrically around said imaginary midline (12; 22).

3. The degradable medical implant according to claim 1 or claim 2, wherein the modulus of elasticity (E) of the essentially two-dimensional mesh (11) varies gradually.

4. The degradable medical implant according to claim 1 or claim 2, wherein the modulus of elasticity (E) of the essentially two-dimensional mesh (21) varies continuously.

5. The degradable medical implant according to any preceding claim, wherein the mesh (11; 21) is synthetic.
